# NEUE EUROPÄISCHE PATENTSCHRIFT

(11) **EP 1 924 144 B2**
(45) Veröffentlichungstag und Bekanntmachung des Hinweises auf die Entscheidung über den Einspruch: **11.10.2017**
(45) Hinweis auf die Patenterteilung: 09.04.2014
(21) Anmeldenummer: 06792607.1
(22) Anmeldetag: 31.07.2006
(51) Int. Cl.: A01N 43/40, A01N 31/02, A01P 1/00

(54) **WUND- UND SCHLEIMHAUTDESINFEKTIONSMITTEL**
WOUND AND MUCOUS MEMBRANE DISINFECTANT
PRODUIT DESINFECTANT POUR BLESSURES ET MUQUEUSES

(30) Priorität: 26.08.2005 DE 102005041730; 09.12.2005 DE 102005058978
(43) Veröffentlichungstag der Anmeldung: 28.05.2008
(73) Patentinhaber: Bode Chemie GmbH, 22525 Hamburg (DE)
(72) Erfinder: KRUG, Barbara, 20259 Hamburg (DE); DABEK, Sven, 22523 Hamburg (DE); MÜLLER, Kai-Martin, 22527 Hamburg (DE); RUDOLF, Marco, 4053 Basel (CH); PIETSCH, Hanns, 20148 Hamburg (DE); OSTERMEYER, Christiane, 22559 Hamburg (DE)
(74) Vertreter: Keller, Günter
(86) Internationale Anmeldenummer: PCT/EP2006/064836
(87) Internationale Veröffentlichungsnummer: WO 2007/023066

(56) Entgegenhaltungen:
- EP-A- 1 683 416
- EP-A1- 0 161 426
- EP-A1- 0 411 315
- WO-A-03/067988
- WO-A2-98/20095
- WO-A2-02/069874
- DE-C1- 3 925 540
- US-A- 4 206 215
- US-A- 4 542 125
- US-A- 4 839 372
- US-A- 5 516 510

## Beschreibung

Die vorliegende Erfindung betrifft ein wässriges Wund- und Schleimhautdesinfektionsmittel auf der Basis von Octenidindihydrochlorid, welches weitere Inhaltsstoffe aus der Gruppe Ethanol, Propan-1-ol, enthält. Das Desinfektionsmittel gemäß der vorliegenden Erfindung hat einen pH-Wert von 5 bis 7 und ist frei von Phenoxyethanol, Phenoxypropanol, Phenoxyisopropanol und organischen Säuren.

Ein Wund- und Schleimhautdesinfektionsmittel sollte in erster Linie bestimmte mikrobiologische Anforderungen erfüllen, welche beispielsweise in F.-A. Pitten, H.-P. Werner, A. Kramer, "A standardized test to assess the impact of different organic challenges on the antimicrobial activity to antiseptics", Journal of Hospital Infection (2003) 55, 108 - 115 beschrieben werden. Allerdings wird auch auf die Wund-, Haut- bzw. Schleimhautverträglichkeit eines entsprechenden Desinfektionsmittels immer höheren Wert gelegt.

Die meisten der bisher üblichen Wirkstoffe weisen hinsichtlich ihrer Verträglichkeit erhebliche Defizite auf. Jod und PVP-Jod verursachen bei Überempfindlichkeit häufig Allergien, außerdem wird die Haut stark verfärbt und Jod penetriert insbesondere aus alkoholischen Lösungen durch die Haut und stärker noch durch die Schleimhaut, was bei empfindlichen Personen zur Hyperthyreose und auch zum Jodismus führen kann. Bei PVP-Jod sind diese Nebenwirkungen zwar geringer, jedoch ebenfalls manifest.

Chlorhexidin und seine Salze sind weltweit neben Jod und PVP-Jod sowie den Alkoholen die wichtigsten Wirkstoffe in der Antiseptik, obwohl diese Verbindungen in toxikologischer Hinsicht kritisch beurteilt werden. Chlorhexidin ist im Ames-Test und im DNA-Repair-Test positiv. Beide Ergebnisse deuten auf ein mutagenes Potenzial hin. Die Abbauprodukte 4-Chloranilin und 4-Chlorphenylisocyanat haben eine große Affinität zur Haut und reichern sich dort bei häufigem Gebrauch an. Triclosan, ein chloriertes Phenol, penetriert sehr stark durch die Haut und ist ein potenzieller Dioxinbildner.

Octenidindihydrochlorid ist als Wirkstoff in Schleimhaut- und Wundantiseptika bekannt und lässt sich durch die nachstehenden Grenzstrukturformeln beschreiben:

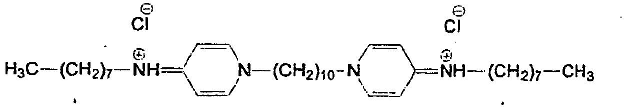

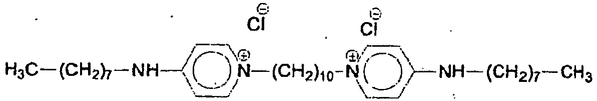

Der Rohstoff Octenidindihydrochlorid besitzt eine gute mikrobizide Wirksamkeit bei relativ guter Verträglichkeit. In der DE-39 25 540-C1 wird ein wässriges Schleimhautantiseptikum beschrieben, welches zur Erhöhung der Wirksamkeit neben Octenidindihydrochlorid Phenoxyethanol und/oder Phenoxypropanol enthält. Ein derartiges Präparat ist beispielsweise unter dem Namen "Octenisept" im Handel und wird vielfach in der Gynäkologie und Andrologie benutzt. Allerdings haben neuere Untersuchungen ergeben, dass die Kombination von Octenidindihydrochlorid und Phenoxyethanol eine hohe Cytotoxizität aufweist, so dass bei der Anwendung auf offenen Wunden beträchtliche Bedenken angebracht sind.

In der WO-02/069874-A1 entsprechend der DE-101 09 925-A1 werden daher Wund- und Schleimhautdesinfektionsmittel beschrieben, die statt der oben genannten Kombination neben Octenidindihydrochlorid nun Ethanol und eine physiologisch verträgliche organische Säure enthalten. Als organische Säure werden beispielhaft Milchsäure, Glycolsäure, Malonsäure, Bernsteinsäure, Äpfelsäure, Weinsäure oder Zitronensäure genannt. Der pH-Wert dieser Lösungen liegt bei 2,5 bis 3,0. Saure Zubereitungen in diesem sehr niedrigen Bereich können bei nicht zu häufigem Gebrauch durchaus verträglich sein, stellen jedoch bei länger andauernder Anwendung eine Noxe dar.

Grundsätzlich besitzen alle mikrobiziden Wirkstoffe ein gewisses Reizpotenzial, auf das die Schleimhaut besonders empfindlich reagiert.

Es war daher Aufgabe der Erfindung, ein Wund- und Schleimhautdesinfektionsmittel auf der Basis von Octenidindihydrochlorid zu entwickeln, welches einerseits die mikrobiziden Anforderungen - besonders hinsichtlich der Wirksamkeit gegenüber Candida albicans - erfüllt, andererseits aber einen gegenüber den Formulierungen des Standes der Technik günstigeren pH-Wert und damit eine bessere Wund-, Haut- bzw. Schleimhautverträglichkeit aufweist.

Völlig überraschend und für den Fachmann nicht vorhersehbar werden diese Aufgaben - insbesondere hinsichtlich der Wirksamkeit gegenüber Candida albicans - gelöst durch ein wässriges Wund-und Schleimhautdesinfektionsmittel, gemäß den Ansprüchen.

Ein erfindungsgemäßes Wund- und Schleimhautdesinfektionsmittel besteht aus
a) 0,05 bis 0,15 Gew.-% Octenidindihydrochlorid,
b) 3,0 bis 12,0 Gew.-% Ethanol,
c) 0,3 bis 1,0 Gew.-% Glycerin (85 Gew.-%),
d) Wasser auf 100 Gew.-%
oder
a) 0,05 bis 0,15 Gew.-% Octenidindihydrochlorid,
b) 2,0 bis 6,0 Gew.-% Propan-1-ol,
c) 0,3 bis 1,0 Gew.-% Glycerin (85 Gew.-%)
d) Wasser auf 100 Gew.-%.

Zusätzlich können die erfindungsgemäßen Wund- und Schleimhautdesinfektionsmittel weitere Stoffe wie Farbstoffe, Parfüms, Emulgatoren, Lösungsvermittler, pH-Regulatoren, Verdickungsmittel und/oder Tenside enthalten. Hierfür kommen erfindungsgemäß bevorzugt nur solche Stoffe in Frage, die kein eigenes oder ein sehr geringes zusätzliches Reizpotenzial aufweisen.

Diese Anforderung gilt selbstverständlich in besonderem Maß für die Tenside und Emulgatoren. Insbesondere geeignet für eine Anwendung im Sinne der vorliegenden Erfindung sind dementsprechend Tenside und Emulgatoren, die im Erythrocyten-Test (RBC-Test = red blood cell-Test) Code 11035 und/oder im HET-CAM-Test, (hen's egg test-chorioaallantoic membrane) Code 11087 kein oder ein sehr geringes Reizpotenzial aufweisen. Hinsichtlich einer genaueren Abstufung zwischen den Tensiden und Emulgatoren sei dabei u.a. auf die einschlägige Literatur zu dem obigen Thema verwiesen.

Erfindungsgemäß besonders vorteilhaft sollten - wenn überhaupt - nur kleine Mengen von sehr gut schleimhautverträglichen Tensiden und Emulgatoren verwendet werden, da ansonsten u. a. eine zu starke Reizung der Schleimhäute stattfinden kann.

Geeignete und dementsprechend vorteilhaft im Sinne der vorliegenden Erfindung zu verwendende Tenside sind zum Beispiel ethoxyliertes Glycerylpalmitat (INCi-Bezeichnung: PEG-200 Hydrogenated Glyceryl Palmate), ethoxyliertes Sorbitan Laurat (INCi: PEG-80 Sorbitan Laurate), ethoxyliertes Glyceryl Isostearat (INCi: PEG-90 Glyceryl Isostearate), ethoxylierter Laurylalkohol (tNCi: Laureth-2) oder auch Betaine wie z.B. Cocamidopropyl Betaine (INCi), wobei sich eine Einsatzkonzentration zwischen 0 und 1,0 Gew.-% als vorteilhaft erwiesen hat. Bezüglich der Emulgatoren sei exemplarisch auf die ethoxylierten Fettalkohole oder auch Fettsäureester (INCi: Ceteareth-20, Glyceryl Stearate) verwiesen.

Die erfindungsgemäßen Wund- und Schleimhautdesinfektionsmittel sind klare Lösungen mit einem pH-Wert von 5 bis 7. Dieser pH-Wert ergibt sich zum Beispiel in der Rezeptur quasi "von selbst". In anderen Fällen kann es erforderlich sein, den pH-Wert entsprechend einzustellen. Dafür haben sich erfindungsgemäß Natronlauge oder Phosphorsäure bewährt.

Die Wund- und Schleimhautdesinfektionsmittel können vorteilhaft im Sinne der vorliegenden Erfindung auch Verdickungsmittel enthalten. Es sind erfindungsgemäß bevorzugt synthetische Polymere auf Polyacrylsäure Basis oder natürliche Verdicker, wie Xanthan Gum oder modifizierte natürliche Verdickertypen, wie z.B. Cellulose Derivate zu verwenden.

Das erfindungsgemäße Wund- und Schleimhautdesinfektionsmittel kann in verschiedenen Applikationsformen eingesetzt werden, z.B. als solches (d. h. in Form einer wäßrigen Lösung), als Gel oder auch als Tränkungslösung für Tücher und/oder Pflaster. Das Wund- und Schleimhautdesinfektionsmittel im Sinne der vorliegenden Erfindung kann ferner bevorzugt aber auch die wässrige Phase einer Emulsion darstellen, die darüber hinaus als Ölphase solche Lipide enthält, die als besonders wund-und schleimhautverträglich bekannt sind, wie z. B. natürliche Öle oder modifizierte Öle und Fette (z. B. Mandelöl, hydrogeniertes Rizinusöl).

Gegenstand der vorliegenden Erfindung sind daher auch O/W- oder W/O-Emulsionen - beispielsweise in Form einer Creme, Lotion oder eines Spray - welche das wässrige Schleimhautdesinfektionsmittel umfassen.

Die vorliegende Erfindung wird durch die nachfolgenden Beispiele näher beschrieben.

### Beispiele:

| **Rohstoffe** | **1** | **2** | **3** | **4** | **5** | **6** | **7** | **8** | **9** | **10** | **11** | **12** |
|---|---|---|---|---|---|---|---|---|---|---|---|---|
| Octenidindihydrochlorid | 0,1 | 0,1 | 0,1 | 0,05 | 0,15 | 0,1 | 0,1 | 0,1 | 0,05 | 0,2 | 0,1 | 0,1 |
| Ethanol (99 Gew.-%) | 10,0 | | | 12,0 | 3,0 | 7,0 | 7,0 | | | | | 15,0 |
| Propan-1-ol | | 4,0 | | | | | 3,0 | | 15,0 | | | |
| Propan-2-ol | | | 4,0 | | 2,0 | | | | | 1,0 | | |
| Undecylenamidopropyltrimonium Methosulfat | | | | 2,1 | | | | 4,1 | | 1,1 | | |
| Natriumhydroxymethylglycinat | | | | | 0,05 | | | 0,5 | | | | |
| 3-(4-Chlorphenoxy)-1,2-propandiol | | | | | | | | | | 0,03 | | 0,3 |
| Glycerin (85 Gew.-%) | 0,5 | 0,5 | 0,5 | 1,5 | | 3,0 | 1,5 | 0,5 | 0,5 | | 0,5 | |
| Pentan-1,2-diol | | | | | 1,0 | | | | 0,5 | 0,2 | | 3,0 |
| NaOH-Lsg. | var. | var. | var. | var. | var. | var. | var. | var. | var. | var. | var. | var. |
| Phosphorsäurelsg. | var. | var. | var. | var. | var. | var. | var. | var. | var. | var. | var. | var. |
| Wasser | ad 100 | ad 100 | ad 100 | ad 100 | ad 100 | ad 100 | ad 100 | ad 100 | ad 100 | ad 100 | ad 100 | ad 100 |

| | | | | | | | | | | | | |
|---|---|---|---|---|---|---|---|---|---|---|---|---|
| Mengen in Gew.-% Die Beispiele 1 und 2 sind erfindungsgemäß. Bei den Beispielen 3 bis 12 handelt es sich um Referenzbeispiele. | | | | | | | | | | | | |

Es wurden Verträglichkeitsuntersuchungen an skarifizierter Haut durchgeführt. Dabei zeigte sich, dass die erfindungsgemäßen Wund- und Schleimhautdesinfektionsmittel besser verträglich sind als die Zubereitungen des Standes der Technik.

## Patentansprüche

1. Wässriges Wund- und Schleimhautdesinfektionsmittel,
**dadurch gekennzeichnet, dass** es aus
a) 0,05 bis 0,15 Gew.% Octenidindihydrochlorid,
b) 3,0 bis 12,0 Gew.-% Ethanol,
c) 0,3 bis 1,0 Gew.% Glycerin (85 Gew.-%),
d) Wasser auf 100 Gew.-% besteht oder
**dass** es aus
a) 0,05 bis 0,15 Gew.-% Octenidindihydrochlorid,
b) 2,0 bis 6,0 Gew.% Propan-1-ol,
c) 0,3 bis 1,0 Gew.% Glycerin (85 Gew.-%),
d) Wasser auf 100 Gew.-% besteht,
wobei das Mittel frei von Phenoxyethanol, Phenoxypropanol, Phenoxyisopropanol, und organischen Säuren ist.

2. Desinfektionsmittel nach Anspruch 1, **dadurch gekennzeichnet, dass** der pH-Wert des Mittels zwischen 5 und 7 liegt.

3. Desinfektionsmittel nach einem der vorhergehenden Ansprüche, **dadurch gekennzeichnet, dass** es weitere Zusatzstoffe wie Tenside, Emulgatoren, Lösungsvermittler, Farbstoffe, pH-Regulatoren und/oder Verdickungsmittel enthält.

4. Desinfektionsmittel nach Anspruch 3, **dadurch gekennzeichnet, dass** als Tenside ethoxyliertes Glycerylpalmitat oder ethoxyliertes Sorbitan Laurat oder ethoxyliertes Glyceryl isostearat oder ethoxylierter Laurylalkohol oder Cocamidopropyl betain (jeweils einzeln oder in beliebigen Mischungen miteinander) verwendet werden.

5. Desinfektionsmittel nach einem der Ansprüche 3 oder 4, **dadurch gekennzeichnet, dass** der Gehalt der Tenside (eine oder mehrere Verbindungen) aus dem Bereich von 0 bis 1,0 Gew.-% gewählt wird.

6. Desinfektionsmittel nach einem der vorherigen Anspruche, **dadurch gekennzeichnet, dass** der Gehalt der Emulgatoren (eine oder mehrere Verbindungen) aus dem Bereich von 0 bis 5,0 Gew.% gewählt wird.

7. Desinfektionsmittel nach Anspruch 3, **dadurch gekennzeichnet, dass** als Verdickungsmittel polyacrylische, natürliche oder modifizierte natürliche Verdicker (einzeln oder in beliebigen Mischungen miteinander) verwendet werden.

8. Desinfektionsmittel nach einem der vorherigen Anspruche, **dadurch gekennzeichnet, dass** zum Einstellen des pH-Wertes je nach Erfordernis entweder Natronlauge oder Phosphorsäure verwendet werden.

9. Desinfektionsmittel nach einem der vorherigen Anspruche, **dadurch gekennzeichnet, dass** es in Form einer Flüssigkeit, eines Gels, einer Creme, einer Lotion, eines Sprays oder Tränkungslosung für Tücher und/oder Pflaster Anwendung findet.

## Claims

1. Aqueous wound and mucous membrane disinfectant,
**characterized in that** said disinfectant comprises
a) 0.05 to 0.15% by weight of octenidine dihydrochloride,
b) 3.0 to 12.0% by weight of ethanol,
c) 0.3 to 1.0% by weight of glycerol (85% by weight),
d) made up to 100% by weight with water, or
that it comprises
a) 0.05 to 0.15% by weight of octenidine dihydrochloride,
b) 2.0 to 6.0% by weight of propan-1-ol,
c) 0.3 to 1.0% by weight of glycerol (85% by weight),
d) made up to 100% by weight with water,
wherein the disinfectant is free from phenoxyethanol, phenoxypropanol, phenoxyisopropanol and organic acids.

2. Disinfectant according to claim 1, **characterized in that** the pH of the agent is between 5 and 7.

3. Disinfectant according to any of the preceding claims, **characterized in that** said disinfectant comprises further additives such as surfactants, emulsifiers, solubilizers, dyes, pH regulators and/or thickeners.

4. Disinfectant according to claim 3, **characterized in that** the surfactants used are ethoxylated glyceryl palmitate or ethoxylated sorbitan laurate or ethoxylated glyceryl isostearate or ethoxylated lauryl alcohol or cocamidopropyl betaine (each individually or in any mixture with one another).

5. Disinfectant according to any one of claims 3 or 4, **characterized in that** the surfactant content (one or more compounds) is selected in the range from 0 to 1.0% by weight.

6. Disinfectant according to any of the preceding claims, **characterized in that** the emulsifier content (one or more compounds) is selected in the range from 0 to 5.0% by weight.

7. Disinfectant according to claim 3, **characterized in that** the thickeners used are polyacrylic, natural or modified natural thickeners (individually or in any mixture with one another).

8. Disinfectant according to any of the preceding claims, **characterized in that** either aqueous sodium hydroxide solution or phosphoric acid are used to adjust the pH according to requirements.

9. Disinfectant according to any of the preceding claims, **characterized in that** said disinfectant is used in the form of a liquid, a gel, a cream, a lotion, a spray or impregnating solution for cloths and/or plasters.

## Revendications

1. Désinfectant aqueux pour plaies et muqueuses,
**caractérisé en ce qu'**il contient
a) 0,05 à 0,15 % en poids de dichlorhydrate d'octénidine,
b) 3,0 à 12,0 % en poids d'éthanol,
c) 0,3 à 1,0 % en poids de glycérol (à 85 % en poids)
d) d'eau en complément à 100 % en poids, ou
qu'il contient
a) 0,05 à 0,15 % en poids de dichlorhydrate d'octénidine,
b) 2,0 à 6,0 % en poids de propan-1-ol,
c) 0,3 à 1,0 % en poids de glycérol (à 85 % en poids),
d) d'eau en complément à 100 % en poids,
le désinfectant étant exempt de phénoxyéthanol, phénoxypropanol, phénoxyisopropanol et d'acides organiques.

2. Désinfectant selon la revendication 1, **caractérisé en ce que** le pH du désinfectant est compris entre 5 et 7.

3. Désinfectant selon l'une quelconque des revendications précédentes, **caractérisé en ce qu'**il contient d'autres additifs tels que des tensioactifs, des émulsifiants, des tiers-solvants, des colorants, des régulateurs de pH et/ou des épaississants.

4. Désinfectant selon la revendication 3, **caractérisé en ce qu'**on utilise comme tensioactifs du palmitate de glycéryle éthoxylé ou du laurate de sorbitanne éthoxylé ou de l'isostéarate de glycéryle éthoxylé ou de l'alcool laurylique éthoxylé ou de la bétaïne de cocamidopropyle (chacun/e individuellement ou en mélanges quelconques entre eux).

5. Désinfectant selon l'une quelconque des revendications 3 et 4, **caractérisé en ce qu'**on choisit la teneur en tensioactifs (un ou plusieurs composés) dans la plage allant de 0 à 1,0 % en poids.

6. Désinfectant selon l'une quelconque des revendications précédentes, **caractérisé en ce qu'**on choisit la teneur en émulsifiants (un ou plusieurs composés) dans la plage allant de 0 à 5,0 % en poids.

7. Désinfectant selon la revendication 3, **caractérisé en ce qu'**on utilise comme épaississants des épaississants polyacryliques, naturels ou naturels modifiés (individuellement ou en mélanges quelconques entre eux).

8. Désinfectant selon l'une quelconque des revendications précédentes, **caractérisé en ce que** pour l'ajustement du pH on utilise au besoin soit une solution d'hydroxyde de sodium, soit de l'acide phosphorique.

9. Désinfectant selon l'une quelconque des revendications précédentes, **caractérisé en ce qu'**on l'utilise sous forme d'un liquide, d'un gel, d'une crème, d'une lotion, d'un spray ou d'une solution d'imprégnation pour lingettes et/ou pansements.
